# EUROPEAN PATENT APPLICATION

(11) **EP 4 180 729 A1**
(43) Date of publication of application: **17.05.2023**
(21) Application number: 20943410.9
(22) Date of filing: 20.07.2020
(51) Int. Cl.: F24F 3/16, A61G 10/00

(54) **AIR INTAKE MECHANISM AND NEGATIVE-PRESSURE SYSTEM**

(30) Priority: 30.06.2020 CN 202010614445
(71) Applicant: Shenzhen Relicare Medical Co., Ltd., Shenzhen, Guangdong 518000 (CN)
(72) Inventor: HE, Wei, Shenzhen, Guangdong 518000 (CN); RAO, Tao, Shenzhen, Guangdong 518000 (CN); HUANG, Yutai, Shenzhen, Guangdong 518000 (CN); ZHU, Guoyuan, Shenzhen, Guangdong 518000 (CN)
(74) Representative: HGF
(86) International application number: PCT/CN2020/102975
(87) International publication number: WO 2022/000588

(57) **Abstract**

Embodiments of the present disclosure provide an air intake mechanism and a negative pressure system (10). The air intake mechanism includes a first air intake end (225), a first air outlet end (226), and a first passage (227) provided between the first air intake end (225) and the first air outlet end (226), a first air driving unit (228) is provided on the first passage (227), a first filter assembly is provided between the first air intake end (225) and the first air driving unit (228), and a second filter assembly is provided between the first air outlet end (226) and the first air driving unit (228).

## Description

### TECHNICAL FIELD

The present disclosure relates to the technical field of protection, and in particular, to an air intake mechanism and a negative pressure system.

### BACKGROUND

Harmful substances are often transmitted through the air, in order to prevent them from spreading, the harmful substances are generally isolated from the outside world in the way of isolation.

The traditional negative pressure system prevents the leakage of harmful substances by forming differential pressure. Since the air intake mechanism supplying air to the negative pressure system lacks a filtering function, the cleanliness of the intake air cannot be guaranteed, thus, there is a risk of further deterioration of the air quality inside the negative pressure system.

### SUMMARY

The main objective of the present disclosure is to provide a negative pressure system when preventing the harmful substances from transmitting with the air, which aims to solve the technical problem that the conventional negative pressure system cannot filter the intake air.

In order to achieve the above objective, the first technical means of the present disclosure are as follows.

The present disclosure provides an air intake mechanism, including: a first air intake end, a first air outlet end, and a first passage provided between the first air intake end and the first air outlet end, a first air driving unit is provided on the first passage, a first filter assembly is provided between the first air intake end and the first air driving unit, and a second filter assembly is provided between the first air outlet end and the first air driving unit.

In some embodiments of the air intake mechanism, an inner wall of the first passage is provided with an embedded portion for fixing the first filter assembly, and a first purification assembly is provided between the first air driving unit and the first filter assembly.

In order to achieve the above objective, the second technical means of the present disclosure are as follows.

The present disclosure further provides a negative pressure system, including:
a closed structure that is unfolded to form a chamber, the closed structure includes an opening-closing mechanism for communicating or separating the chamber and an external space of the closed structure;
a support device for unfolding the closed structure;
a detection device for detecting an inner air pressure of the closed structure; and
an air exchanger for exchanging after purifying an air inside and outside the closed structure,
the air exchanger includes an air outlet mechanism, a purification mechanism and the air intake mechanism as described above, the purification mechanism includes the first purification assembly and the second purification assembly, the second purification assembly is for purifying the air passing through the air outlet mechanism;
the air intake mechanism is for intaking air into the closed structure, the air outlet mechanism is for discharging the air in the closed structure to adjust an air pressure inside the closed structure to be lower than an air pressure outside the closed structure.

In some embodiments of the negative pressure system, the opening-closing mechanism is a door structure for people to enter and exit.

In some embodiments of the negative pressure system, the support device includes a plurality of support mechanisms;
the adjacent support mechanisms are connected as a whole when closed to each other, the closed structure is unfolded when the adjacent support mechanisms are away from each other.

In some embodiments of the negative pressure system, the support mechanism includes a body, at least one of the bodies is provided with the air intake mechanism, and at least one of the bodies is provided with the air output mechanism.

In some embodiments of the negative pressure system, at least one of the bodies is provided with an operation console, the operation console is configured to control an airflow passing through the air intake mechanism and the airflow passing through the air outlet mechanism according to an air pressure value detected by the detection device.

In some embodiments of the negative pressure system, a telescopic rod is provided on a top of the support mechanism, and the telescopic rod is accommodated in the support mechanism.

In some embodiments of the negative pressure system, the negative pressure system further including: a telescopic connecting frame for driving the adjacent support mechanisms to be close to each other and away from each other.

Implementing the embodiments of the present disclosure will have the following beneficial effects:
by providing a first filter assembly between the first air intake end and the first air driving unit and providing a second filter assembly between the first air outlet end and the first air driving unit, the air intake mechanism ensures the cleanliness of the air after passing through the air intake mechanism, and avoids further deterioration of the air quality in the chamber supplied with air by the air intake mechanism.

### BRIEF DESCRIPTION OF THE DRAWINGS

In order to more clearly illustrate the technical solutions in the embodiments of the present disclosure or in the prior art, the following briefly introduces the accompanying drawings that need to be used in the description of the embodiments or the prior art. Obviously, the drawings in the following description are only some embodiments of the present disclosure. For those of ordinary skill in the art, other drawings can also be obtained based on the structures shown in these drawings without any creative effort.

In which,
FIG. 1 is a front view of a negative pressure system being unfolded according to an embodiment of the present disclosure.
FIG. 2 is an axial view of the negative pressure system shown in FIG. 1.
FIG. 3 is a top view of the negative pressure system shown in FIG. 1.
FIG. 4 is an axial view of a closed structure in the negative pressure system shown in FIG. 1.
FIG. 5 is an axial view of a contracted support device according to an embodiment.
FIG. 6 is a schematic view of the enlarged structure of portion A in FIG. 5.
FIG. 7 is a schematic view of the enlarged structure of portion B in FIG. 5.
FIG. 8 is a schematic view of the support device in the negative pressure system shown in FIG. 1 being unfolded along the X direction.
FIG. 9 is a schematic view of the support device in the negative pressure system shown in FIG. 1 being unfolded along the Y direction.
FIG. 10 is a schematic view of stretching out of a telescopic rod in the negative pressure system shown in FIG. 1.
FIG. 11 is a schematic structural view of a first support mechanism in the negative pressure system shown in FIG. 1.
FIG. 12 is a schematic structural view of a third support mechanism in the negative pressure system shown in FIG. 1.
FIG. 13 is a schematic structural view of the first air driving unit or second air driving unit in the negative pressure system shown in FIG. 1.
FIG. 14 is a schematic view of a connecting bar in the negative pressure system shown in FIG. 1.
FIG. 15 is an axial view of an internal structure of a body of the first support mechanism in an embodiment.
FIG. 16 is an axial view from another angle of view of the internal structure of the body of the first support mechanism shown in FIG. 15.
FIG. 17 is a front view of the internal structure of the body of the first support mechanism shown in FIG. 15.
FIG. 18 is a cross sectional view along C-C in FIG. 17.
FIG. 19 is a top view of the internal structure of the body of the first support mechanism shown in FIG. 15.

### DETAILED DESCRIPTION OF THE EMBODIMENTS

The technical solutions of the embodiments of the present disclosure will be described in more detail below with reference to the accompanying drawings. It is obvious that the embodiments to be described are only some rather than all of the embodiments of the present disclosure. All other embodiments obtained by persons skilled in the art based on the embodiments of the present disclosure without creative efforts shall fall within the scope of the present disclosure.

The negative pressure system 10 provided in the embodiment of the present disclosure is used to prevent harmful substances from spreading through the air, the harmful substances include harmful gases, liquids, solid particles, mycoplasma and pathogens that can be transmitted through the air, the harmful substances further include aerosols containing mycoplasma and or pathogens. Of course, in other embodiments of the present disclosure, the negative pressure system 10 can also be used to prevent the transmission of harmful substances through other media, or the transmission of other non-harmful substances, or be applied to other situations that require differential pressure, which will not be uniquely limited here.

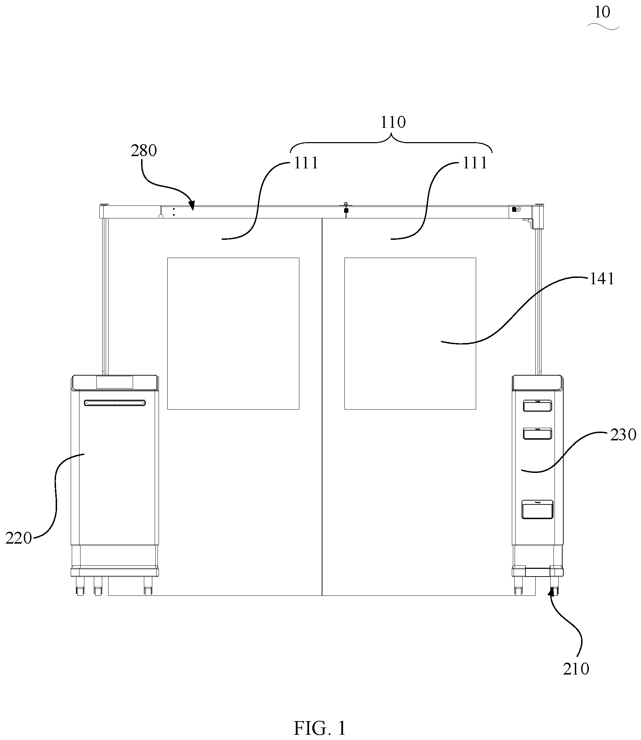

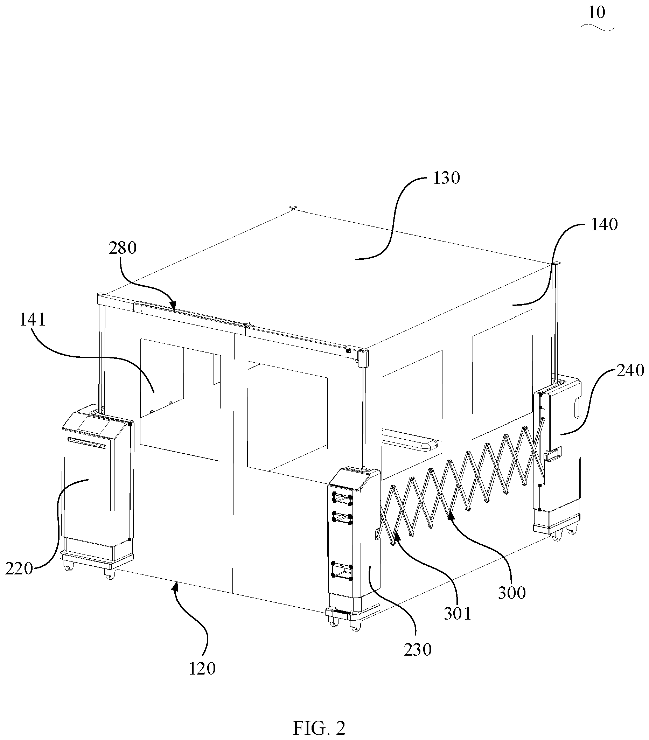

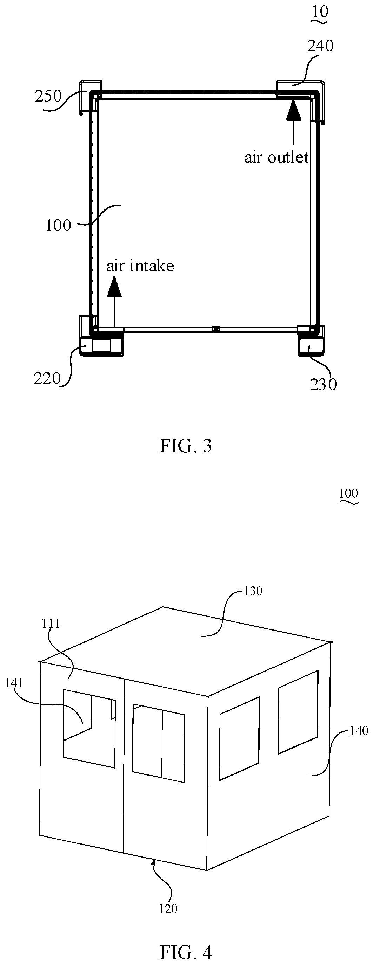

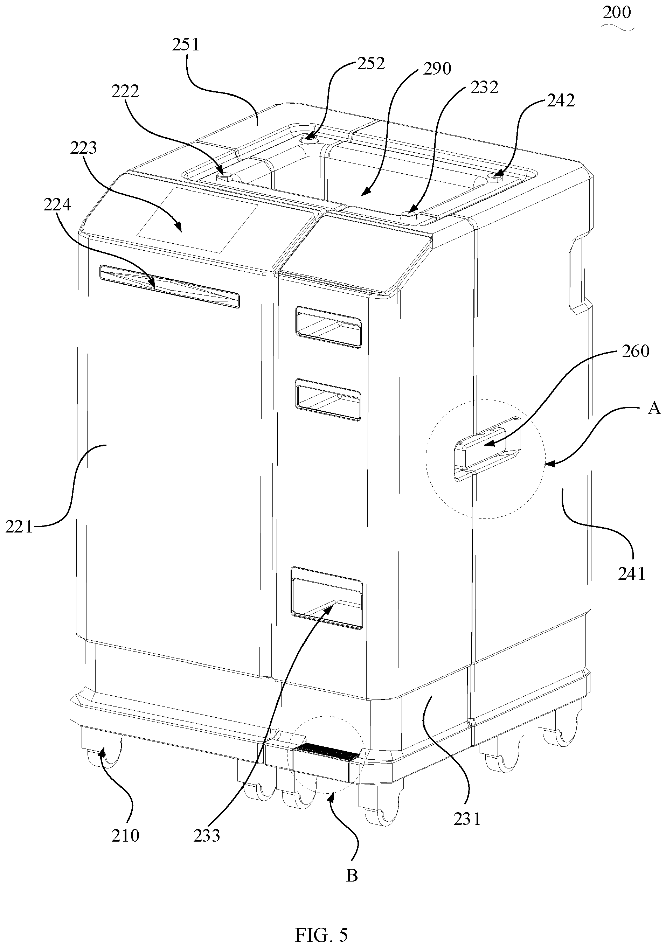

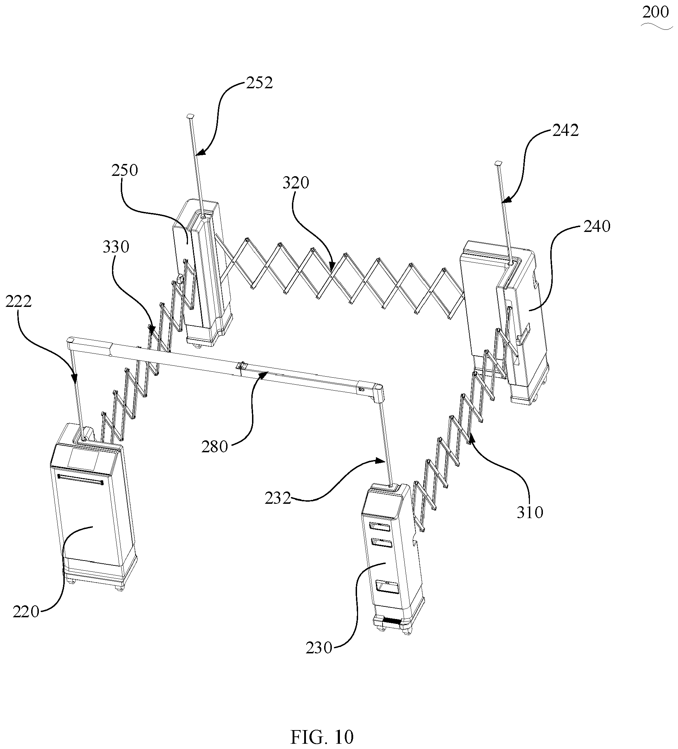

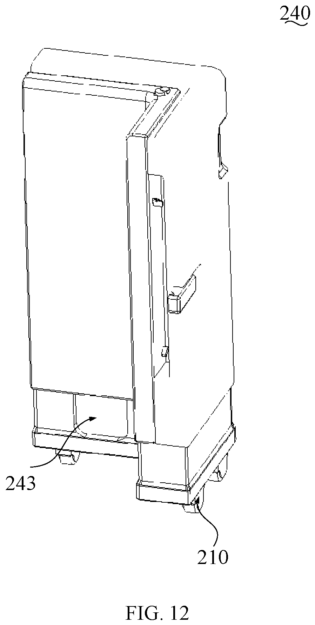

In combination with FIG. 1 to FIG. 14, the negative pressure system 10 provided by the present disclosure is described. A negative pressure system 10 includes: a closed structure 100, a support device 200, a detection device and an air exchanger. The closed structure 100 is a flexible structure or a folded structure that can be composed of a plurality of spliced panels, the folded structure can be folded to reduce its volume for portability, and it has a certain space after unfolded. Further, the closed structure 100 can be unfolded to form a chamber, and the closed structure 100 includes an opening-closing mechanism 110, which is for communicating or separating the chamber and the external space of the closed structure 100. The above-mentioned negative pressure system 10 adopts a closed structure 100, which can be unfolded to form a chamber. The chamber is communicated or separated from the external space through the opening-closing mechanism 110. When the opening-closing mechanism 110 is closed, the chamber can be completely isolated from the external space to effectively prevent the spread of harmful substances.

With reference to FIG. 1 to FIG. 4, the closed structure 100 is a flexible structure, and the shape of the closed structure 100 after unfolding can be a cuboid, cylinder, hemispheroid or other shapes that can form a certain space. In this embodiment, the closed structure 100 is in a shape of cuboid. Specifically, the closed structure 100 includes a bottom subsection 120, a top subsection 130, and a side subsection 140 between the bottom subsection 120 and the top subsection 130, and the bottom subsection 120, the top subsection 130, and the side subsection 140 is unfolded to form a chamber. In this embodiment, the opening-closing mechanism 110 includes two opening-closing portions 111, and the two opening-closing portions 111 are closed by means of magnetic attraction or mechanical connection. A sealing structure is further provided between the two opening-closing portions 111 to ensure that the chamber is isolated from the external space when closed. The side subsection 140 also has a viewing window 141 made of a transparent medium.

In this embodiment, the opening-closing mechanism 110 is a door structure for people to enter and exit, and the opening-closing portion 111 is a flexible structure. For example, the opening-closing portion 111 is a flexible curtain, by folding the two opening-closing portions 111 away from each other, a passage into and out of the chamber is formed. It can be understood that, in other embodiments, the top of the opening-closing portion 111 and the side away from the other opening-closing portions 111 are integrally connected with the closed structure 100, and the bottom of the opening-closing portion 111 is detachably connected with the closed structure 100, which can also be realized by means of magnetic attraction or mechanical connection, and a sealing structure is provided between the bottom of the opening-closing portion 111 and the closed structure 100 to ensure that the chamber is isolated from the external space when closed. That is, an L-shaped connecting seam is formed between the adjacent opening-closing portions 111 and the bottom of the opening-closing portion 111, the opening-closing portion 111 can be lifted up along the L-shaped connecting seam to enter and exit the chamber. When the opening-closing mechanism 110 is closed, the closed structure 100 is a fully sealed structure, and the gas can only be exchanged through the gas exchanger, which ensures the absolute isolation of harmful substances from the outside world.

Further, the support device 200 can unfold the closed structure 100. Specifically, the support device 200 includes a plurality of support mechanisms. The adjacent support mechanisms can be connected as one when approaching to each other, and can be connected in a magnetic or mechanical way. The closed structure 100 can be unfolded when adjacent support mechanisms move away. Further, a telescopic rod is provided on the top of the support mechanism, and the telescopic rod can be accommodated in the support mechanism. When the adjacent support mechanisms are away, the height of the support mechanism cannot realize that when the closed structure 100 is unfolded in its axial direction, the telescopic rod is stretched out and locked firmly to increase the height of the support mechanism, and the closed structure 100 is unfolded and shown in a final shape, the closed structure 100 is connected with the support mechanism and the telescopic rod through a connecting piece. A plurality of rollers 210 are provided at the bottom of the support mechanism to make it convenient for the adjacent support mechanisms to be close or to move away. The roller 210 is provided with a locking portion, which can lock the roller 210 after the support mechanism moves to a preset position, so as to prevent the support mechanism from moving. The existence of the roller 210 facilitates the overall movement of the support device 200, and the closed structure 100 is a flexible structure, so that the negative pressure system 10 has strong portability as a whole when it is contracted.

In this embodiment, the support device 200 includes a first support mechanism 220, a second support mechanism 230, a third support mechanism 240, and a fourth support mechanism 250. The first support mechanism 220 and the second support mechanism 230 are connected through a magnetic piece, the third support mechanism 240 and the fourth support mechanism 250 are also connected through the magnetic piece, and an outer side of the magnetic piece is wrapped with an elastic element to reduce the impact caused by the collision during the connecting, the first support mechanism 220 and the fourth support mechanism 250 are connected through a lock catch 260, and the second support mechanism 230 and the third support mechanism 240 are also connected through the lock catch 260. The lock catch 260 includes a connecting block 261 and a connection groove, the connecting block 261 can be rotated relative to the first support mechanism 220 or second support mechanism 230, a connecting protrusion 2611 is formed on the connecting block, and is matched with the connecting groove located on the third support mechanism 240 or fourth support mechanism 250, the connecting block 261 and the connecting groove can be connected by means of magnetic attraction or mechanical connection. Further, the magnetic piece is an electromagnetic piece, and the support device 200 is provided with a touching switch 270, through which the power of the magnetic piece can be turned off and on, so that the magnetic attraction force of the magnetic piece is disappeared and formed. After the magnetic attraction force is formed, the elastic piece is squeezed to form an elastic force, when the magnetic attraction force disappears, with the effect of the elastic force, the first support mechanism 220 and the second support mechanism 230 connected through the lock catch 260 can be bounced off each other, and the third support mechanism 240 and the fourth support mechanism 250 can also be bounced off each other, the first support mechanism 220 and the second support mechanism 230 as a whole are conveniently unfolded by the inertia, and the third support mechanism 240 and the fourth support mechanism 250 as a whole are also conveniently unfolded by the inertia. After the magnetic piece generates magnetic attraction force, touch the touching switch 270 once, the magnetic attraction force is disappeared, and then touch the touching switch 270 again, and the magnetic attraction force is regenerated.

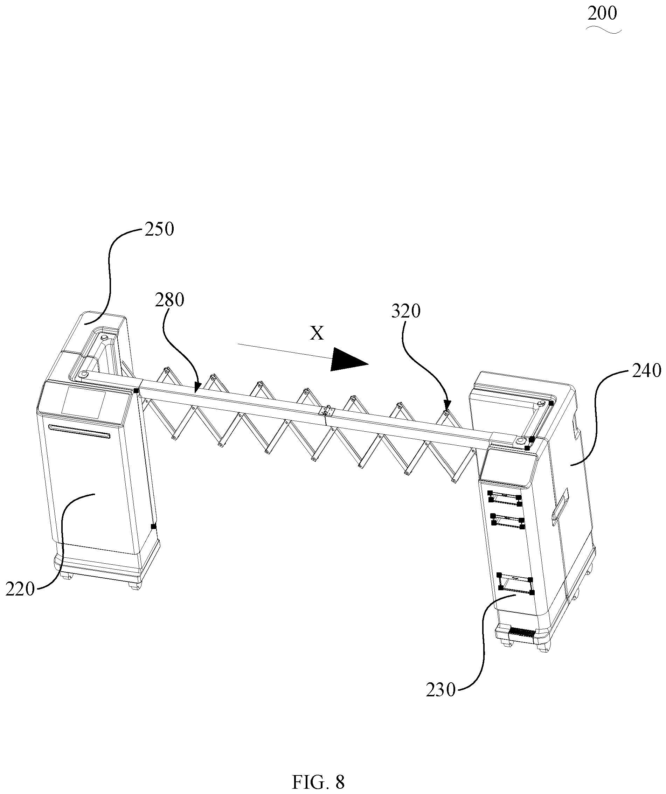

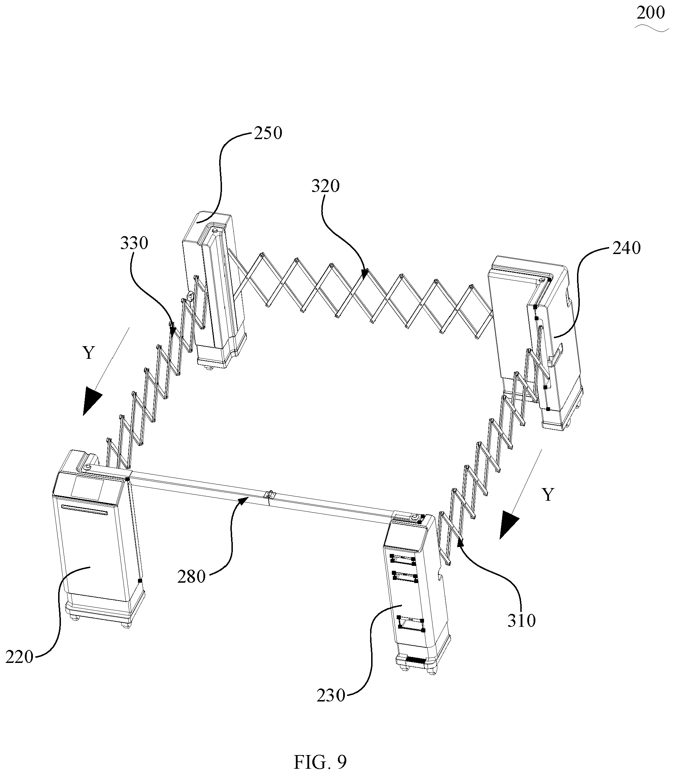

Further, the negative pressure system 10 includes a telescopic connecting frame 300, which is used to drive the adjacent support mechanisms to be close and to move away. The connecting frame 300 includes a first connecting frame 310 connected between the second support mechanism 230 and the third support mechanism 240, a second connecting frame 320 connected between the third support mechanism 240 and the fourth support mechanism 250, and a third connection frame 330 between the fourth support mechanism 250 and the first support mechanism 220, by providing the aforementioned three groups of connection frames 300, the support mechanisms that are close to each other can be away from each other, and the direction and distance away from each other is limited, therefore the closure structure 100 is unfolded. As mentioned above, when the magnetic attraction force disappears, with the effect of the elastic force, the first support mechanism 220 and the second support mechanism 230 connected as a whole through the lock catch 260 and the third support mechanism 240 and the second support mechanism connected as a whole through the lock catch 260 can be bounced off each other, the second connecting frame 320 is extended along the X direction in FIG. 8 to unfold the first support mechanism 220 and the second support mechanism 230 connected as a whole and the third support mechanism 240 and the second support mechanism connected as a whole. After that, the lock catch 260 is opened, the first connecting frame 310 is extended along the Y direction in FIG. 9 to unfold the second support mechanism 230 and the third support mechanism 240, the third connecting frame 330 is extended along the Y direction in FIG. 9 to unfold the first support mechanism 220 and the fourth support mechanism 250, finally, each support mechanism is locked by the rollers 210. In this embodiment, the connecting frame 300 is composed of a plurality of connecting rods 301 hinged head to end, and the two connecting rods 301 at the ends are respectively connected to the corresponding support mechanisms.

Further, the detection device is used to detect an inner air pressure of the closed structure 100. Specifically, the detection device is used to detect the air pressure of the chamber.

Further, the gas exchanger is used to purify and exchange the gas inside and outside the closed structure 100, so as to adjust the air pressure inside the closed structure 100 to be lower than the air pressure outside the closed structure 100. Further, the gas exchanger is a fresh air device with a purification function, and the gas exchanger includes an air intake mechanism, an air outlet mechanism and a purification mechanism, the purification mechanism is used to purify the gas passing through the air intake mechanism and the gas passing through the air outlet mechanism. The air intake mechanism is used to intake air into the closed structure 100, and the air outlet mechanism is used to discharge the gas in the closed structure 100 to adjust the inner air pressure of the closed structure 100 to be lower than the air pressure outside the closed structure 100.

Specifically, the air outlet mechanism is communicated with the chamber, and is used to adjust the air pressure of the chamber to be lower than the air pressure outside the chamber. The air intake mechanism includes a first air intake end 225, a first air outlet end 226, and a first passage provided between the first air intake end 225 and the first air outlet end 226, a first air driving unit 500 is provided on the first passage. The first air intake end 225 is communicated with an input port 501 of the first air driving unit 500, the first air outlet end 226 is communicated with the output port 502 of the first air driving unit 500, and the gas located in the external space of the closed structure 100 is driven through the first air driving unit 500 and can enter the chamber through the first passage. The air outlet mechanism includes a second air inlet end 243, a second air outlet end, and a second passage provided between the second air inlet end 243 and the second air outlet end, the second air driving unit 600 is provided on the second passage, the air inlet end 243 is communicated with an input end 601 of the second air driving unit 600, and the second air outlet end is communicated with an output end 602 of the second air driving unit 600, the gas located in the chamber is driven by the second air driving unit 600 and can be passed through the second passage to exit the chamber. The purification mechanism includes a first purification assembly provided in the first passage and a second purification assembly provided in the second passage. Specifically, the first purification assembly includes a first air purification net and a first connecting piece for fixing the first air purification net in the first passage, the second purification assembly includes a second air purification net and a second connecting piece for fixing the second air purification net in the second passage. Both the first air purification net and the second air purification net are HEPA filter nets, the second air purification net further has the function of sterilization and detoxification, so as to ensure that the discharged gas is free of harmful substances. Further, through the combined effect of the air intake mechanism and the air outlet mechanism, the chamber forms a differential pressure.

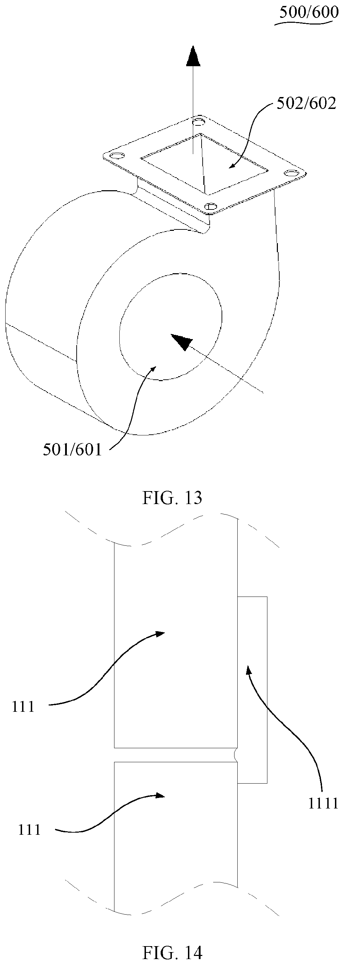

As shown in Figure 14, further, a connecting strip 1111 is provided on the detachable connection between the opening-closing portion 111 and the closed structure 100 and the connection between the adjacent opening-closing portions 111, the connecting stripe 1111 provided on the connection between the adjacent opening-closing portions 111 is fixedly connected with one of the opening-closing portions 111. The connecting strip 1111 is located in the circumferential direction of the opening-closing portion 111 and on a side away from the chamber. Since the inner air pressure of the chamber is lower than the outer air pressure of the chamber, the connecting strip 1111 is adsorbed at each connection to ensure that the chamber is isolated from the external space, the surface of the connecting stripe 1111 is processed to reduce its sliding friction, thereby reducing an opening resistance of the opening-closing portion 111. The connecting strip 1111 can be made of flexible material, which can be deformed with the effect of air pressure, so as to further ensure the isolation of the chamber from the external space.

Further, the support mechanism includes a body, at least one body is provided with an air intake mechanism, and at least one body is provided with an air outlet mechanism. At least one body is provided with an operation console, and the operation console controls an airflow passing through the intake mechanism and the airflow passing through the air outlet mechanism according to an air pressure value detected by the detection device.

Specifically, the first support mechanism 220 includes a first body 221, an air intake mechanism is provided inside the first body 221, a first air outlet end 226 is communicated with the chamber, a roller 210 is provided at the bottom of the first body 221, and a first telescopic rod 222 that can stretch out from its top is accommodated inside the first body 221, an operation console is also provided in the first body 221. The operation console includes a display screen 223, a processing unit, a receiving unit, a sending unit and a report generating unit, the processing unit is for controlling the airflow rate passing through the air intake mechanism and the airflow rate passing through the air outlet mechanism according to the air pressure value detected by the first detecting device. The receiving unit is used to receive external signals, for example, control signals to control the operation of the negative pressure system 10, data signals, including inspection information of infectious patients, and the like. The sending unit is for sending signals to the outside, for example, a running information of the negative pressure system 10, the monitoring data of the infected patients, and the like. The report generating unit is used to generate a paper report and export it from the report outlet 224 of the first body 221. The paper report may include status information of the negative pressure system 10 within a period of time, monitoring data of infected patients or inspection reports of infected patients, etc. The display screen 223 is provided on the top of the first body 221, and is for displaying the air pressure value detected by the first detection device, the airflow rate of the air intake mechanism and the air outlet mechanism, the monitoring data of the infectious patient, or the inspection report of the infectious patient, etc., and the screen 223 has an interactive function. The gas quality inside the closed structure 100 detected by the second detection device 400 can also be displayed in real time through the display screen 223, so that the gas quality inside the closed structure 100 can be known in real time.

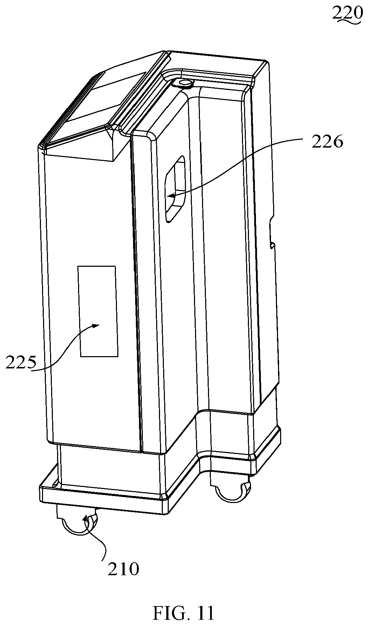

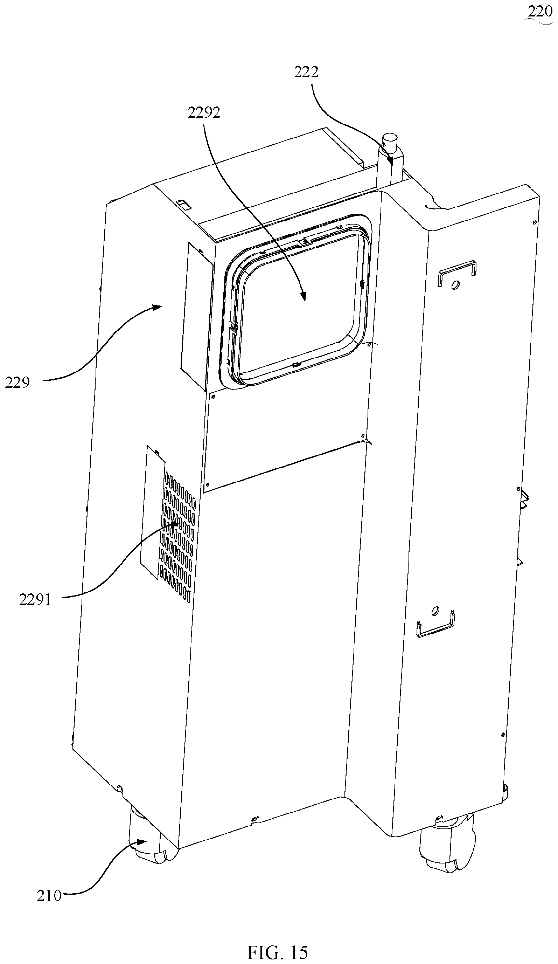

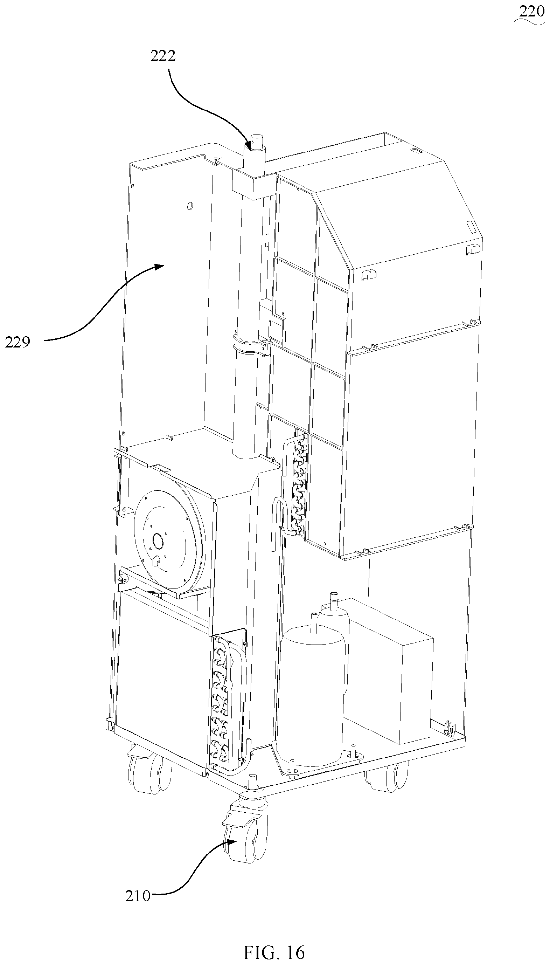

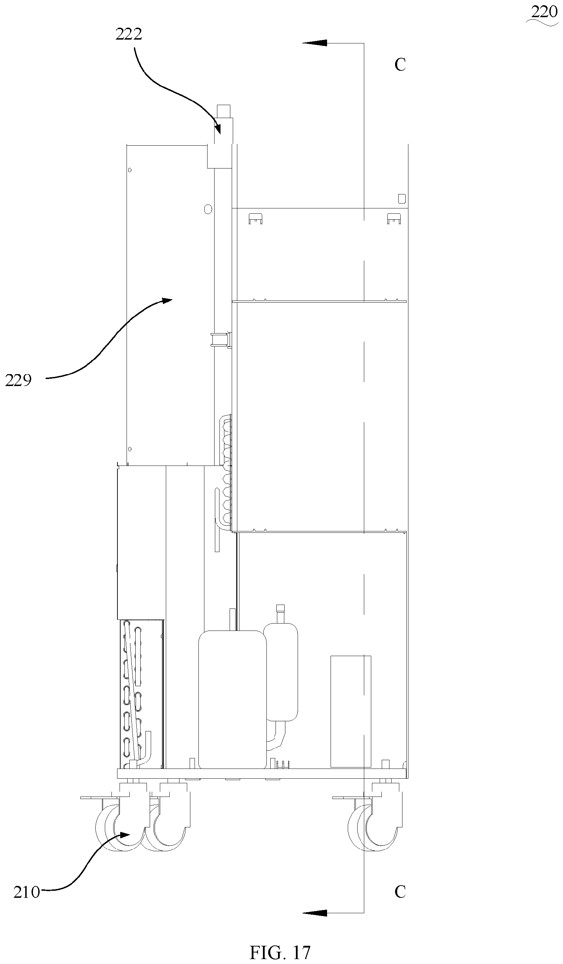

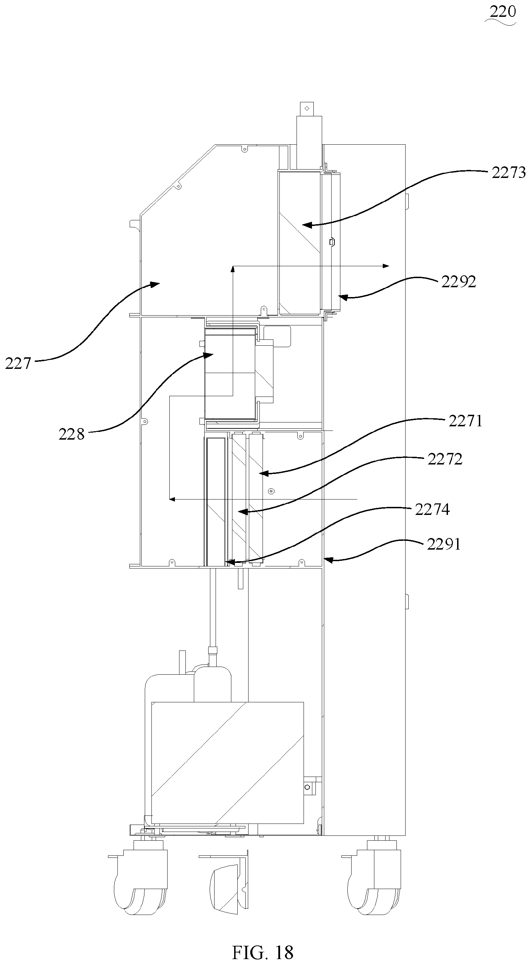

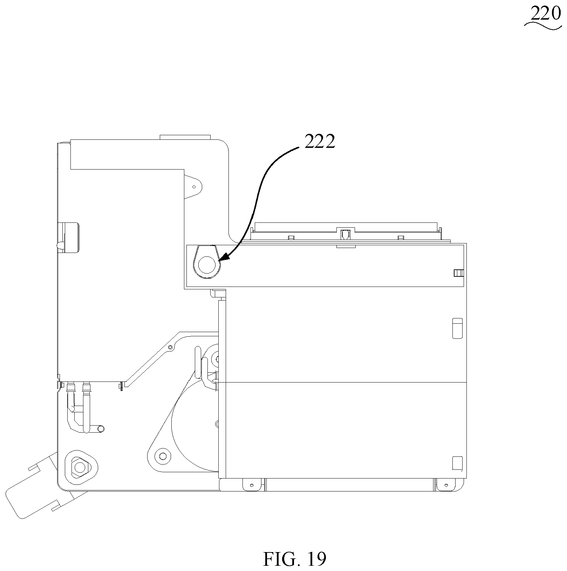

In another embodiment, with reference to FIG. 11 and FIG. 15 to FIG. 19, the air intake mechanism includes a first air intake end 225, a first air outlet end 226, and a first passage 227 provided between the first air intake end 225 and the first air outlet end 226. A first air driving unit 228 is provided on the first passage 227. Further, with reference to FIG. 15 to FIG. 19, a first filter assembly is provided between the first air intake end 225 and the first air driving unit 228. A second filter assembly is provided between the first air outlet 226 and the first air driving unit 228. The inner wall of the first passage 227 is provided with an embedded portion for fixing the first filter assembly. A first purification assembly is provided between the first air driving unit 228 and the first filter assembly. Specifically, the first filter assembly includes a low efficiency filter 2271 and a medium efficiency filter 2272. The inner wall of the first passage 227 is provided with embedded parts for fixing the primary efficiency filter 2271 and the medium efficiency filter 2272. The second filter assembly includes a high efficiency filter 2273. The high efficiency filter 2273 is fixedly connected with the first passage 227. Further, the first purification assembly is located between the first air driving unit 228 and the medium-efficiency filter 2272. The first purification assembly includes a first air purification net 2274 and an ultraviolet lamp. The ultraviolet lamp is for sterilizing the gas passing through the first air purification net 2274. The first air purification net 2274 is fixedly connected with the inner wall of the first passage 227. Further, the first support mechanism 220 includes a first body 221, and an air intake mechanism is provided in the first body 221. The first air intake end 225 and the first air outlet end 226 are located on the first body 221. The air intake mechanism also includes an inner shell 229 located in the first body 221. The first passage 227 forms a first through hole 2291 and a second through hole 2292 on the inner shell 229. The primary efficiency filter 2271 is provided close to the first through hole 2291. The first air intake end 225 communicates with the first through hole 2291. The first air outlet end 226 is communicated with the second through hole 2292. Further, the first through hole 2291 is provided with a mesh plate for preliminary filtration of the gas before entering the inner shell 229 to remove impurities of large size, so as to increase the service life of the first filter assembly and the first purification assembly. Further, the first air outlet end 226 is sleeved on the second through hole 2292. The first air outlet 226 is communicated with the chamber, the bottom of the first body 221 is provided with a roller 210, the first body 221 contains a first telescopic rod 222 that can extend from the top, and the first body 221 is also provided with an operation console, the operation console includes a display screen 223, a processing unit, a receiving unit, a sending unit and a report generating unit, the processing unit is for controlling the airflow rate passing through the air intake mechanism and the airflow rate passing through the air outlet mechanism according to the air pressure value detected by the first detecting device. The receiving unit is used to receive external signals, for example, control signals to control the operation of the negative pressure system 10, data signals, including inspection information of infectious patients, and the like. The sending unit is for sending signals to the outside, for example, a running information of the negative pressure system 10, the monitoring data of the infected patients, and the like. The report generating unit is used to generate a paper report and export it from the report outlet 224 of the first body 221. The paper report may include status information of the negative pressure system 10 within a period of time, monitoring data of infected patients or inspection reports of infected patients, etc. The display screen 223 is provided on the top of the first body 221, and is for displaying the air pressure value detected by the first detection device, the airflow rate of the air intake mechanism and the air outlet mechanism, the monitoring data of the infectious patient, or the inspection report of the infectious patient, etc., and the screen 223 has an interactive function. The gas quality inside the closed structure 100 detected by the second detection device 400 can also be displayed in real time through the display screen 223, so that the gas quality inside the closed structure 100 can be known in real time.

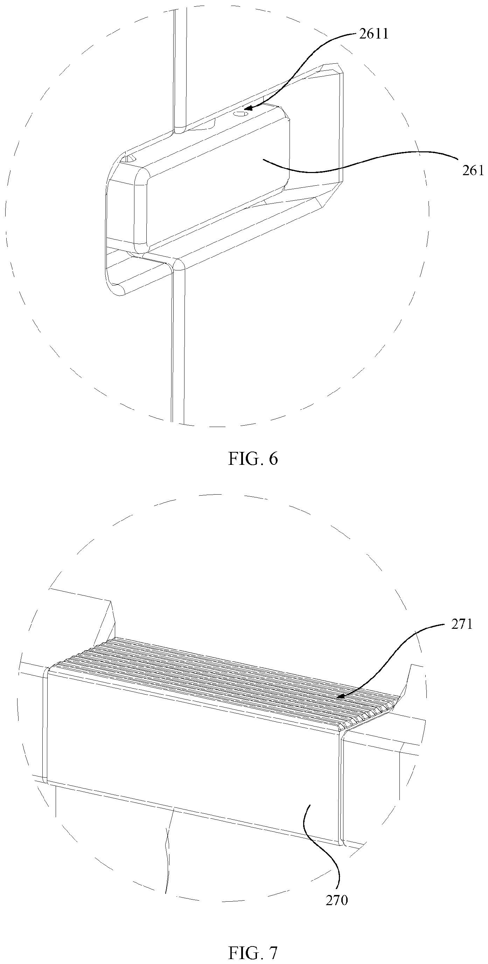

Further, the second support mechanism 230 includes a second body 231, the bottom of the second body 231 is provided with a roller 210, and the second body 231 is accommodated with a second telescopic rod 232 extending from the top thereof. The second body 231 is also formed with a storage slot 233 for storing items. As shown in FIG. 7, the touching switch 270 is provided on the second body 231, and is a foot-touching switch, on which an antiskid portion 271 is provided and can be rotated relative to the second body 231. As shown in FIG. 1 to FIG. 3 and FIG. 8 to FIG. 10, the opening-closing mechanism 110 is provided between the first support mechanism 220 and the second support mechanism 230. In order to drive the opening-closing mechanism 110 to open and close, a cross beam 400 is provided between the first telescopic rod 222 and the second telescopic rod 232, and the cross beam 400 is provided with a driving mechanism for driving the opening-closing mechanism 110 to open and close.

Further, the third support mechanism 240 includes a third body 241, an air outlet mechanism is provided inside the third body 241, a second air intake end 243 is communicated with the chamber, a roller 210 is provided at the bottom of the third body 241, and the third telescoping rod 242 that can stretch out from its top is accommodated inside the third body 241. The first air outlet end 226 and the second air intake end 243 are provided diagonally, which is conducive to the rapid diffusion of the gas entering the chamber. For example, for patients who need to inhale oxygen, air with higher oxygen content can be passed through the first passage. When the chamber needs to be sterilized, the disinfecting air can be introduced from the first passage; when the harmful substances in the chamber need to be removed or reacted, the corresponding air can be introduced from the first passage.

Further, the fourth support mechanism 250 includes a fourth body 251, and a backup battery may be provided in the fourth body 251 to ensure that the negative pressure system 10 can still operate when the external power supply to the negative pressure system 10 is stopped. The bottom of the fourth body 251 is provided with a roller 210, and a fourth telescopic rod 252 extending from the top thereof is accommodated by the fourth body 251.

When the support device 200 is contracted, the first support mechanism 220, the second support mechanism 230, the third support mechanism 240 and the fourth support mechanism 250 can be enclosed to form an accommodation space 290, and the support mechanism 200 can be accommodated in the accommodating space 290 after the contraction of the closed structure 100 and the cross beam 400 is folded, which improves the portability of the negative pressure system 10 when contracted.

The above are only some embodiments of the present disclosure, and do not limit the scope of the present disclosure thereto. Therefore, equivalent transformations made according to the claims of the present disclosure are still included in the scope of the present disclosure.

## Claims

1. An air intake mechanism, **characterized by** comprising:
a first air intake end (225);
a first air outlet end (226); and
a first passage (227) provided between the first air intake end and the first air outlet end,
wherein a first air driving unit (228) is provided on the first passage (227), a first filter assembly is provided between the first air intake end (225) and the first air driving unit (228), and a second filter assembly is provided between the first air outlet end (226) and the first air driving unit (228).

2. The air intake mechanism of claim 1, wherein an inner wall of the first passage (227) is provided with an embedded portion for fixing the first filter assembly, and a first purification assembly is provided between the first air driving unit (228) and the first filter assembly.

3. A negative pressure system (10), **characterized by** comprising:
a closed structure (100) that is unfolded to form a chamber,
wherein the closed structure (100) comprises an opening-closing mechanism (110) for communicating or separating the chamber and an external space of the closed structure (100);
a support device (200) for unfolding the closed structure (100);
a detection device for detecting an inner air pressure of the closed structure (100); and
an air exchanger for exchanging after purifying an air inside and outside the closed structure (100),
wherein the air exchanger comprises an air outlet mechanism, a purification mechanism and the air intake mechanism of claim 2, the purification mechanism comprises the first purification assembly and the second purification assembly, the second purification assembly is for purifying the air passing through the air outlet mechanism;
the air intake mechanism is for intaking air into the closed structure (100), the air outlet mechanism is for discharging the air in the closed structure (100) to adjust an air pressure inside the closed structure (100) to be lower than an air pressure outside the closed structure.

4. The negative pressure system (10) of claim 3, wherein the opening-closing mechanism (110) is a door structure for people to enter and exit.

5. The negative pressure system (10) of claim 3, wherein the support device (200) comprises a plurality of support mechanisms;
the adjacent support mechanisms are connected as a whole when closed to each other, the closed structure (100) is unfolded when the adjacent support mechanisms are away from each other.

6. The negative pressure system (10) of claim 5, wherein the support mechanism comprises a body, at least one of the bodies is provided with the air intake mechanism, and at least one of the bodies is provided with the air output mechanism.

7. The negative pressure system (10) of claim 5, wherein at least one of the bodies is provided with an operation console, the operation console is configured to control an airflow passing through the air intake mechanism and the airflow passing through the air outlet mechanism according to an air pressure value detected by the detection device.

8. The negative pressure system (10) of claim 5, wherein a telescopic rod is provided on a top of the support mechanism, and the telescopic rod is accommodated in the support mechanism.

9. The negative pressure system (10) of claim 5, further comprising:
a telescopic connecting frame (300) for driving the adjacent support mechanisms to be close to each other and away from each other.
